# FASCICULE DE BREVET EUROPEEN

(11) **EP 0 913 198 B1**
(45) Date de publication et mention de la délivrance du brevet: **13.04.2005**
(21) Numéro de dépôt: 98402678.1
(22) Date de dépôt: 27.10.1998
(51) Int. Cl.: B01J 37/02, C10G 45/00

(54) **Procédé de préparation de catalyseurs utilisables dans les réactions de transformation de composés organiques**
Verfahren zur Herstellung von Katalysatoren zur Verwendung in Umwandlungsreaktionen von organischen Verbindungen
Process for preparation of catalysts useful for organic compounds convertion reactions

(30) Priorité: 31.10.1997 FR 9713684
(43) Date de publication de la demande: 06.05.1999
(73) Titulaire: Institut Français du Pétrole, 92852 Rueil-Malmaison Cedex (FR)
(72) Inventeur: Basset, Jean-Marie, 69300 Caluir (FR); Candy, Jean-Pierre, 69300 Caluire (FR); Didillon, Blaise, 92500 Rueil Malmaison (FR); Le Peltier, Fabienne, 92500 Rueil Malmaison (FR); Clause, Olivier, 78400 Chatou (FR); Bentahar, Fatima, 69100 Villeurbanne (FR)

(56) Documents cités:
- EP-A- 0 623 384
- FR-A- 2 594 711
- US-A- 4 548 918
- US-A- 4 645 752
- US-A- 4 737 262

## Description

La présente invention concerne un nouveau procédé de préparation d'un catalyseur renfermant au moins un support, au moins un métal du groupe VIII de la classification périodique des éléments, et au moins un élément additionnel M choisi parmi le germanium, l'étain, le plomb, le gallium, l'indium et le thallium. Ce catalyseur peut aussi contenir un autre métal choisi dans le groupe constitué par les métaux alcalins et/ou un métalloïde tel que le soufre et/ou tout autre élément chimique tel qu'un halogène ou un composé halogéné.

Les brevets et publications démontrant que l'addition de promoteurs à un métal de base améliore la qualité des catalyseurs sont fort nombreux. Ces éléments sont ajoutés sous différentes formes telles que sels ou composés organométalliques. On obtient généralement des catalyseurs plus actifs ou plus sélectifs, parfois plus stables que le catalyseur monométallique correspondant. La façon dont ces modificateurs sont introduits n'est pas indifférente car eile conditionne fortement les propriétés du catalyseur.

Ainsi la formulation de catalyseurs utilisés dans les procédés de conversion d'hydrocarbures a fait l'objet d'un très grand nombre d'études. Les catalyseurs métalliques supportés ont été décrits notamment dans les brevets US-A-3 998 900 et FR-A-2 495 605. Ils contiennent une phase métallique à base de platine, modifiés par un métal additionnel M tel que l'étain, supportés sur des oxydes inorganiques réfractaires tels que l'alumine. L'introduction du métal M est avantageusement effectuée à l'aide d'un composé organométallique dudit métal M. Cette méthode d'introduction du métal M a déjà été décrite dans le brevet US 3 531 543 et dans le brevet US 4 548 918 de la demanderesse.

Les procédés déjà cités décrivent la fabrication d'un catalyseur par l'utilisation d'au moins un composé organométallique du métal M. Le métal M est introduit sous la forme d'au moins un composé organométallique choisi dans le groupe formé par les complexes, en particulier les complexes carbonyles, polycétoniques des métaux M et les hydrocarbyls métaux du métal M tels que les alkyles, les cycloalkyles, les aryles, les akylaryles métaux et les arylalkyles métaux.

L'introduction de l'élément additionnel M sous la forme d'un composé organométallique conduit à des catalyseurs plus performants mais nécessite l'emploi d'un solvant organique. Le solvant d'imprégnation décrit selon la technologie du brevet US 4 548 918 est choisi dans le groupe constitué par les solvants organiques oxygénés contenant de 2 à 8 atomes de carbone par molécule, et les hydrocarbures paraffiniques, naphténiques ou aromatiques contenant essentiellement de 6 à 15 atomes de carbone par molécule, et les composés organiques oxygénés halogénés contenant de 1 à 15 atomes de carbone par molécule. Ces solvants peuvent être utilisés seuls ou en mélange entre eux.

On a maintenant découvert dans la présente invention qu'il est possible de préparer ces catalyseurs particulièrement performants avec une introduction du métal M sous la forme d'un complexe organométallique soluble dans un solvant aqueux. Ceci représente un progrès considérable de facilité de mise en oeuvre de la fabrication du catalyseur. En effet, l'usage de quantités industrielles de solvants organiques présente d'importants inconvénients en termes de sécurité (inflammabilité, toxicité) et en termes de coûts.

Le support du catalyseur selon l'invention comporte au moins un oxyde réfractaire qui est généralement choisi parmi les oxydes de métaux des groupes IIA, IIIA, IIIB, IVA ou IVB, de la classification périodique des éléments tels que par exemple les oxydes de magnésium, d'aluminium, de silicium, de titane, de zirconium, de thorium pris seuls ou en mélange entre eux ou en mélange avec des oxydes d'autres éléments de la classification périodique. On peut aussi utiliser le charbon. On peut aussi utiliser des zéolithes ou tamis moléculaires de type X, Y, mordénite, faujasite, ZSM-5, ZSM-4, ZSM-8, ainsi que les mélanges d'oxydes de métaux des groupes IIA, IIIA, IIIB, IVA et IVB avec du matériau zéolithique.

Pour les réactions de transformations d'hydrocarbures, le support préféré est l'alumine, dont la surface spécifique est avantageusement comprise entre 5 et 400 m² par gramme, de préférence entre 50 et 350 m² par gramme. Les supports préférés utilisés dans le domaine de la transformation de fonctions organiques sont la silice, le charbon et l'alumine.

Le catalyseur selon l'invention, renferme outre un support :
a) au moins un métal du groupe VIII choisi parmi l'iridium, le nickel, le palladium, le platine, le rhodium et le ruthénium. Le platine et le palladium sont les métaux préférés pour les réactions de conversion d'hydrocarbures. Le rhodium et le ruthénium sont les métaux préférés pour la transformation de molécules fonctionnelles. Le pourcentage pondéral est choisi par exemple entre 0,1 et 10 % et de préférence entre 0,1 et 5 %.
b) au moins un élément additionnel M choisi dans le groupe constitué par le germanium, l'étain, le plomb, le rhénium, le gallium, l'indium et le thallium. L'étain et le germanium sont les éléments préférés. Le pourcentage pondéral est choisi par exemple entre 0,01 et 10 %, et de préférence entre 0,02 et 5 %. On peut avantageusement dans certains cas utiliser à la fois au moins deux des métaux de ce groupe.

Suivant le domaine d'application, le catalyseur peut contenir éventuellement en plus par exemple de 0,1 à 3 % poids d'un halogène ou composé halogéné. Il peut aussi contenir de 0,1 à 3 % poids d'un métal alcalin ou alcalino-terreux. Il peut aussi éventuellement contenir de 0.01 à 2 % poids d'un élément tel que le soufre.

Le catalyseur peut être préparé par différentes procédures d'imprégnation du support et l'invention n'est pas limitée à une procédure d'imprégnation déterminée. Quand on utilise plusieurs solutions, on peut procéder à des séchages et/ou des calcinations intermédiaires.

L'élément additionnel M peut être introduit lors de l'élaboration du support. Une méthode consiste par exemple à malaxer la poudre humide de support avec les précurseurs du catalyseur et à mettre ensuite en forme et sécher.

On peut introduire le métal du groupe VIII, le métal additionnel M, éventuellement l'halogène ou le composé halogéné, éventuellement le métal alcalin ou alcalino-terreux, éventuellement le métalloïde, simultanément ou successivement, dans n'importe quel ordre. Selon l'invention, la mise en contact de l'élément organométallique M est caractérisée en ce qu'il est introduit dans un solvant aqueux.

Dans une autre méthode, le métal additionnel M, peut-être introduit lors de la synthèse du support selon une technique de type Sol-Gel. Par exemple, pour un support contenant de l'alumine, un gel mixte métal M-alumine peut être obtenu en hydrolysant avec une solution aqueuse d'un composé organométallique du métal M une solution organique de Al(OR')₃ dans un solvant tel ROH ou R'OH. R et R' peuvent désigner un groupement alkyl de type méthyl, éthyl, isopropyl, n-propyl, butyl, voire un groupement plus lourd, tel le n-hexyl. Le solvant alcoolique doit être déshydraté de façon poussée avant l'introduction de l'alcoolate d'aluminium. Après l'hydrolyse, un traitement thermique du gel obtenu opéré à une température comprise entre 200 et 800°C, de préférence entre 300 et 700°C et de manière encore plus préférée entre 400 et 500°C, permet d'assurer la réaction complète du composé organométallique hydrosoluble du métal M avec le gel, ce qui entraîne la formation de l'oxyde mixte Al₂O₃-MOₓ.

Dans une autre méthode le métal M peut être ajouté à un sol d'alumine. Le brevet US 3 929 683 décrit l'introduction de l'étain sous forme de sel, par exemple SnCl₂ dans un sol d'alumine. Selon la présente invention, il est possible d'ajouter un composé organométallique hydrosoluble de métal M à un hydrosol d'alumine, obtenu par exemple en précipitant à pH 4-5 une solution acide de AlCl₃, puis de favoriser la réaction du composé du métal M avec l'hydrosol d'alumine, par exemple sous l'effet de la chaleur ou d'une base.

Le précurseur de l'élément M peut être choisi, sans que cette liste soit limitative, dans le groupe des composés halogénés, des hydroxydes. des oxydes, des carbonates, et des carboxylates de composés organométalliques de l'élément M. Ces composés comprennent au moins une liaison carbone-M. Le précurseur de l'élément M peut être aussi choisi parmi les composés de formule générale (R1)ₓ M (R2)_{y} avec x+y=valence du métal M et où R1 est choisi dans le groupe des fonctions alkyles, cycloalkyles, aryles, alkylaryles et arylalkyles et R2 est une fonction de la forme CₐH_{b}R'_{c} où R' représente une fonction hydroxyde, carboxylate, PO₃H ou SO₃H.

Dans une technique préférée de préparation selon l'invention, le catalyseur est obtenu par imprégnation du support, à l'aide d'une solution aqueuse ou organique d'au moins un composé de métal du groupe VIII, le volume de la solution étant de préférence en excès par rapport égal volume de rétention du support ou égal à ce volume. Le support imprégné est ensuite filtré, éventuellement lavé à l'eau distillée puis séché et calciné sous air habituellement entre 110 et environ 500°C, puis ensuite réduit sous hydrogène à une température habituellement comprise entre environ 200 et environ 600°C et de préférence entre environ 300 et environ 500°C. Le produit obtenu est alors imprégné par une solution aqueuse d'au moins un composé d'étain, de germanium, de plomb, de rhénium, de gallium, d'indium ou de thallium. D'une manière particulièrement avantageuse, on utilise une solution aqueuse d'un composé carboxylate d'étain. Le volume de la solution aqueuse est de préférence égal au volume de rétention du support et de manière encore plus préférée en excès par rapport à ce volume. La valeur de la concentration d'au moins un métal M dans la solution aqueuse est choisie avantageusement entre 0,01 et 25 mmol/l et de manière préférée entre 0,5 et 20 mmol/l et de manière encore plus préférée entre 0,5 et 15 mmol/l. La valeur de pH de la solution aqueuse est choisie avantageusement entre 10 et 14 et de manière préférée entre 10 et 12.

Après avoir laissé le contact entre le support imprégné du métal du groupe VIII et la solution aqueuse contenant au moins un composé de l'élément M pendant plusieurs heures, le produit est filtré, éventuellement lavé à l'eau puis séché. On termine, selon cette méthode, par une réduction entre 300 et 600°C, de préférence en effectuant un balayage sous hydrogène pendant plusieurs heures. Il est aussi possible de terminer, après l'étape de séchage par une calcination entre 300 et 600°C sous balayage d'air.

Dans une autre technique selon l'invention, le catalyseur est obtenu par imprégnation d'une solution aqueuse d'au moins un composé dudit métal M, le volume de la solution étant de préférence égal au volume de rétention du support et de manière encore plus préférée en excès par rapport à ce volume. D'une manière particulièrement avantageuse, on utilise une solution aqueuse d'un composé carboxylate d'étain. La valeur de la concentration d'au moins un métal M dans la solution aqueuse est choisie avantageusement entre 0,01 et 25 mmol/l et de manière préférée entre 0,5 et 20 mmol/l et de manière encore plus préférée entre 0,5 et 15 mmol/l. La valeur de pH de la solution aqueuse est choisie avantageusement entre 10 et 14 et de manière préférée entre 10 et 12. Après avoir laissé le contact entre le solide et la solution d'imprégnation pendant plusieurs heures, le produit est ensuite séché. On termine habituellement par une calcination entre 300 et 600°C, de préférence en effectuant un balayage d'air durant plusieurs heures. Le solide obtenu est ensuite imprégné à l'aide d'une solution aqueuse ou organique d'au moins un composé de métal du groupe VIII, le volume de la solution étant de préférence en excès par rapport au volume de rétention du support ou égal à ce volume. Après quelques heures de mise en contact, le produit obtenu est ensuite séché puis calciné sous air entre 300 et 600°C, de préférence en effectuant un balayage d'air durant plusieurs heures.

Avant utilisation on réduit le catalyseur sous hydrogène par exemple entre 20 et 600°C afin d'obtenir une phase métallique active. La procédure de ce traitement consiste par exemple en une montée lente de la température sous courant d'hydrogène jusqu'à la température maximale de réduction, comprise par exemple entre 20 et 600°C, et de préférence entre 90 et 500°C, suivie d'un maintien pendant par exemple durant 1 à 6 heures à cette température.

Cette réduction pouvant être effectuée aussitôt après une calcination, ou plus tard chez l'utilisateur. Il est aussi possible de réduire directement le produit séché directement chez l'utilisateur.

Il est aussi possible d'effectuer la réduction préalable du composé de métal du groupe VIII en solution par des molécules organiques à caractère réducteur tels que l'acide formique. On peut alors introduire le composé de l'élément additionnel M simultanément ou successivement. Le solide peut être alors directement utilisé lorsque la réaction catalytique nécessite un solvant aqueux, ce qui est en particulier applicable dans le domaine de transformation de fonctions organiques. Une autre possibilité consiste à filtrer, puis sécher le catalyseur obtenu. Il peut être alors calciné puis réduit dans les conditions décrites ci-dessus. Il est aussi possible d'effectuer la réduction directement à partir du produit séché.

Le catalyseur préparé selon l'invention peut être utilisé dans un procédé de transformation d'hydrocarbures intervenant dans le domaine du raffinage et de la pétrochimie. Il peut aussi être utilisé dans un procédé du domaine de la chimie.

Les exemples qui suivent illustrent l'invention sans toutefois en limiter la portée.

### EXEMPLE 1

On prépare 2 catalyseurs A et B renfermant chacun 0.7 % poids de platine et 0.15 % poids d'étain. Le support est une silice Degussa de surface spécifique de 200 m² par gramme.

### Catalyseur A (comparatif)

Le support est calciné à 500 °C sous air sec pendant 5 heures puis mis en contact avec une solution ammoniacale (1 N) durant 15 heures sous bullage d'azote. Le dépôt de platine est alors réalisé par introduction d'une solution d'hydroxyde de platine tétramine. La suspension est ensuite filtrée, lavée à l'eau distillée et séchée à 110 °C sous débit d'azote. On réduit ensuite 3 grammes du solide contenant 0.7 % poids de platine sous débit d'hydrogène durant 4 heures à 450°C. Le catalyseur est ensuite chargé dans un réacteur contenant de l'heptane sous hydrogène. Le tétrabutylétain est lors injecté à 20°C. Après 24 heures de réaction à la température ambiante sous atmosphère d'hydrogène, le solide est filtré, lavé à l'heptane, puis séché à 80°C. Il est ensuite réduit sous courant d'hydrogène à 550 °C durant 4 heures.

### Catalyseur B (selon l'invention)

Le catalyseur B est préparé à partir de 3 grammes du solide à 0.7 % poids de platine, réduit à 450°C sous débit d'hydrogène durant 4 heures. Le catalyseur est ensuite chargé sous hydrogène dans un réacteur contenant 50 cm³ d'une solution aqueuse ammoniacale (pH 11) contenant 4,5 mg d'étain sous la forme d'acétate de tributyl étain (Bu₃SnOC(O)CH₃). Après 24 heures de mise en contact sous atmosphère d'hydrogène, le mélange réactionnel est filtré, lavé, puis séché à 80°C. Il est ensuite réduit sous courant d'hydrogène à 550 °C durant 4 heures.

### Catalyseur C (selon l'invention)

Le support est préparé par hydrolyse par une solution aqueuse d'acétate de tributyl étain (Bu₃SnOC(O)CH₃) d'une solution d'isopropylate d'aluminium Al(OiPr)3 dans l'isopropanol, suivie d'un traitement thermique à 450°C. Le dépôt de platine est ensuite obtenu par imprégnation à sec d'acide hexachloroplatinique à raison de 0,7% poids de platine rapporté au support calciné. Après calcination à 450°C pendant une durée de 12h le catalyseur est réduit sous courant d'hydrogène à 500°C durant 4 heures.

### EXEMPLE 2

Les catalyseurs A, B et C sont soumis à un test de déshydrogénation d'isobutane dans un réacteur tubulaire isotherme. 1 g de catalyseur sont réduit à 550 °C durant 2 heures sous un débit de 2 litres par heure d'hydrogène. Après injection de la charge on stabilise la température à 550°C. L'analyse des effluents gazeux se fait en ligne par chromatographie en phase gazeuse.

Les conditions opératoires sont les suivantes :
- charge : iC₄ Air liquide N35
- température : 550 °C
- pression : 0.1 MPa
- H₂/ iC₄ (molaire) : 1
- débit horaire massique iC₄ liquide/ masse de catalyseur: 725 h⁻¹

Les résultats obtenus dans ces conditions sont rapportés dans le tableau 1.

**Tableau 1**

| Catalyseurs | Durée (h) | Activité (a) | Sélectivité isobutène (b) |
|---|---|---|---|
| A | 1 | 20.0 | 99.1 |
| | 3 | 18.4 | 99.1 |
| | 5 | 17.6 | 99.3 |
| | | | |
| B | 1 | 25.0 | 99.2 |
| | 3 | 23.5 | 99.2 |
| | 5 | 23.0 | 99.3 |
| | | | |
| C | 1 | 22.1 | 99.1 |
| | 3 | 18.8 | 99.1 |
| | 5 | 17.8 | 99.3 |

| | | | |
|---|---|---|---|
| (a) (moles. g Pt⁻¹ .s⁻¹ .10³) | | | |
| (b) (% moles) | | | |

Ces résultats montrent bien que les catalyseurs B et C préparés selon l'invention, à partir d'un précurseur organométallique de l'élément M en phase aqueuse, ont des performances catalytiques au moins identiques et mêmes supérieures à celles du catalyseur A préparé selon l'art antérieur à partir du tétrabutylétain en phase organique.

## Revendications

1. Procédé de préparation d'un catalyseur renfermant au moins un support, au moins un métal du groupe VIII de la classification périodique des éléments et au moins un élément additionnel M choisi dans le groupe constitué par le germanium, l'étain, le plomb, le rhénium, le gallium, l'indium, le thallium, ledit procédé étant **caractérisé en ce qu'**on introduit ledit métal M dans un solvant aqueux sous la forme d'au moins un composé organométallique, soluble dans un solvant aqueux, comprenant au moins une liaison carbone-M.

2. Procédé selon la revendication 1 tel qu'on introduit en outre dans le catalyseur au moins un métal alcalin ou alcalino-terreux.

3. Procédé selon l'une des revendications 1 à 2 tel qu'on introduit en outre dans le catalyseur au moins un métalloïde.

4. Procédé selon l'une des revendications 1 à 3 tel qu'on introduit en outre dans le catalyseur au moins un halogène ou un composé halogéné.

5. Procédé selon l'une des revendications 1 à 4 tel que le métal du groupe VIII est choisi parmi l'iridium, le nickel, le palladium, le platine, le rhodium et le ruthénium, de préférence le platine et le palladium.

6. Procédé selon l'une des revendications 1 à 5 tel que l'élément M est choisi parmi le germanium et l'étain.

7. Procédé selon l'une des revendications 1 à 6 tel que le précurseur de l'élément M est choisi dans le groupe des hydroxydes, des composés halogénés, des carbonates, les carboxylates de composés organiques de l'élément M, les composés de formules générales (R1)ₓ M (R2)_{y} avec x+y=valence du métal M et où R1 est choisi dans le groupe des fonctions alkyles, cycloalkyles, aryles, alkylaryles et arylalkyles et R2 est une fonction de la forme CₐH_{b}R'_{c} où R' représente une fonction hydroxyde, carboxylate, PO₃H ou SO₃H.

8. Procédé selon l'une des revendications 1 à 7 tel que le précurseur de l'élément M est choisi dans le groupe des composés halogénés, des hydroxydes, des oxydes, des carbonates, et des carboxylates de composés organométalliques de l'élément M.

9. Procédé selon l'une des revendications 1 à 7 tel que le précurseur de l'élément M est choisi dans le groupe des carboxylates de composés organiques de l'élément M.

10. Procédé selon la revendication 9 tel que le précurseur de l'élément M est l'acétate . de tributyl étain.

11. Procédé de préparation d'un catalyseur selon l'une des revendications 1 à 10 tel que le métal du groupe VIII, l'élément additionnel M, éventuellement l'halogène ou le composé halogéné, éventuellement le métal alcalin ou alcalino-terreux, éventuellement le métalloïde, sont introduits simultanément ou successivement.

12. Procédé de préparation d'un catalyseur selon l'une des revendications 1 à 11 tel que dans n'importe quel ordre :
• on imprègne un support à l'aide d'une solution aqueuse ou organique d'au moins un métal du groupe VIII, on filtre, on sèche, on calcine, on réduit,
• on imprègne le produit obtenu par une solution aqueuse d'au moins un composé de l'élément M additionnel, on filtre, on sèche, on réduit éventuellement, on calcine.

13. Procédé de préparation d'un catalyseur selon l'une des revendications 1 à 12 dans lequel on imprègne le support avec une solution aqueuse d'au moins un composé du métal M, le volume de la solution étant au moins égal au volume de rétention du support, et de préférence supérieur à ce volume.

14. Procédé selon l'une des revendications 1 à 13 tel que la concentration en au moins un métal M dans la solution aqueuse est comprise entre 0,01 et 25 mmol/l.

15. Procédé selon la revendication 14 tel que la concentration en au moins un métal M dans la solution aqueuse est comprise entre 0,5 et 20 mmol/l et de préférence entre 0,5 et 15 mmol/l.

16. Procédé de préparation d'un catalyseur selon l'une des revendications 1 à 15 tel que la valeur de pH de la solution aqueuse d'au moins un composé du métal M est choisie entre 10 et 14 et de manière préférée entre 10 et 12.

17. Procédé de préparation d'un catalyseur selon l'une des revendications 1 à 11 dans lequel on introduit l'élément additionnel M lors de l'élaboration du support.

18. Procédé de préparation d'un catalyseur selon la revendication 17 tel que l'élément additionnel M est introduit lors de la synthèse du support selon une technique de type SOL-GEL.

19. Procédé de préparation d'un catalyseur selon la revendication 18 tel qu'on hydrolyse avec une solution aqueuse d'un composé organométallique de métal M une solution organique d'un composé alcoxy d'un métal du support, dans un solvant alcoolique puis on chauffe à une température comprise entre 200 et 800°C.

20. Utilisation de catalyseurs préparés selon l'une des revendications 1 à 19 dans les réactions de transformation de composés organiques.

21. Utilisation de catalyseurs selon la revendication 20 dans laquelle lesdites réactions sont des réactions de déshydrogénation d'hydrocarbures aliphatiques saturés en hydrocarbures oléfiniques correspondants.

22. Utilisation de catalyseurs selon la revendication 21 telle que lesdits hydrocarbures aliphatiques saturés sont en C3-C4.

## Patentansprüche

1. Herstellungsverfahren eines Katalysators, der wenigstens einen Träger, wenigstens ein Metall der Gruppe VIII des Periodensystems der Elemente und wenigstens ein zusätzliches Element M umfasst, gewählt aus der Gruppe, die besteht aus Germanium, Zinn, Blei, Rhenium, Gallium, Indium, Thallium, wobei das Verfahren **dadurch gekennzeichnet ist, dass** man das Metall M in einem wässrigen Lösungsmittel in Form von wenigstens einer organometallischen, in einem wässrigen Lösungsmittel löslichen Verbindung einführt, die wenigstens eine Kohlenstoff-M-Bindung umfasst.

2. Verfahren nach Anspruch 1, derart, dass man außerdem in den Katalysator wenigstens ein Alkali- oder Erdalkalimetall einführt.

3. Verfahren nach einem der Ansprüche 1 bis 2, derart, dass man außerdem in den Katalysator wenigstens ein Metalloid bzw. Nichtmetall einführt.

4. Verfahren nach einem der Ansprüche 1 bis 3, derart, dass man außerdem in den Katalysator wenigstens ein Halogen oder eine Halogenverbindung einführt.

5. Verfahren nach einem der Ansprüche 1 bis 4, bei dem das Metall der Gruppe VIII gewählt ist unter Iridium, Nickel, Palladium, Platin, Rhodium und Ruthenium, vorzugsweise Platin und Palladium,

6. Verfahren nach einem der Ansprüche 1 bis 5, derart, dass das Element M gewählt ist unter Germanium und Zinn.

7. Verfahren nach einem der Ansprüche 1 bis 6, bei dem der Vorläufer des Elements M gewählt ist aus der Gruppe der Hydroxide, der Halogenverbindungen, der Carbonate, der Carboxylate von organischen Verbindungen des Elements M, den Verbindungen der allgemeinen Formeln (R1)ₓ M (R2)_{y} mit x+y= Valenz des Metalls M und wobei R1 gewählt ist aus der Gruppe der Alkyl-, Cycloalkyl-, Aryl-, Alkylaryl- und Aralkylfunktionen und R2 eine Funktion der Form CₐH_{b}R'_{c}, worin R' eine Hydroxid-, Carboxylat-, PO₃H- oder SO₃H-Funktion darstellt.

8. Verfahren nach einem der Ansprüche 1 bis 7, derart, dass der Vorläufer des Elements gewählt ist aus der Gruppe der Halogenverbindungen, der Hydroxide, der Oxide, der Carbonate und der Carboxylate von organometallischen Verbindungen des Elements M.

9. Verfahren nach einem der Ansprüche 1 bis 7, derart, dass das Element M gewählt ist aus der Gruppe der Carboxylate von organischen Verbindungen des Elements M.

10. Verfahren nach Anspruch 9, derart dass der Vorläufer des Elements M Tributylzinnacetat ist.

11. Verfahren zur Herstellung eines Katalysators nach einem der Ansprüche 1 bis 10, derart, dass das Metall der Gruppe VIII, das Zusatzelement M, gegebenenfalls das Halogen oder die Halogenverbindung, gegebenenfalls das Alkali- oder Erdalkalimetall oder gegebenenfalls das Metalloid bzw. Nichtmetall; gleichzeitig oder aufeinander folgend eingeführt werden.

12. Verfahren zur Herstellung eines Katalysators nach einem der Ansprüche 1 bis 11, derart, dass man in irgendeiner Weise:
- einen Träger mithilfe einer wässrigen organischen Lösung wenigstens eines Metalls der Gruppe VIII imprägniert, man filtriert, man trocknet, man kalziniert, man reduziert,
- man das erhaltene Produkt durch eine wässrige Lösung wenigstens einer Verbindung des Zusatzelements M imprägniert, man filtriert, man trocknet, man reduziert gegebenenfalls, man kalziniert.

13. Herstellungsverfahren eines Katalysators nach einem der Ansprüche 1 bis 12, derart, dass man den Träger mit einer wässrigen Lösung wenigstens einer Verbindung des Metalls M imprägniert, wobei das Volumen der Lösung wenigstens gleich dem Rückhaltevolumen des Trägers ist und vorzugsweise größer als dieses Volumen.

14. Verfahren nach einem der Ansprüche 1 bis 13, derart, dass die Konzentration an wenigstens einem Metall M in der wässrigen Lösung zwischen 0,01 und 25 mmol/l liegt.

15. Verfahren nach Anspruch 14, derart, dass die Konzentration an wenigstens einem Metall M der wässrigen Lösung zwischen 0,5 bis 20 mmol/l und vorzugsweise zwischen 0,5 und 15 mmol/l liegt.

16. Herstellungsverfahren eines Katalysators nach einem der Ansprüche 1 bis 15, derart, dass der pH-Wert der wässrigen Lösung wenigstens einer Verbindung des Metalls M gewählt ist zwischen 10 und 14 und in bevorzugter Weise zwischen 10 und 12.

17. Verfahren zur Herstellung eines Katalysators nach einem der Ansprüche 1 bis 11, derart, dass man das Zusatzelement M bei der Ausarbeitung des Trägers einführt.

18. Verfahren zur Herstellung eines Katalysators nach Anspruch 17, derart dass das Zusatzelement M bei der Synthese des Trägers gemäß einer Technik vom Typ SOL-GEL eingeführt wird.

19. Verfahren zur Herstellung eines Katalysators nach Anspruch 18, derart, dass man mit einer wässrigen Lösung einer organometallischen Verbindung eines Metalls M eine organische Lösung einer Alkoxyverbindung eines Metalls des Trägers in einem alkoholischen Lösungsmittel hydrolysiert und man dann auf eine Temperatur zwischen 200 und 800°C heizt.

20. Verwendung von Katalysatoren, hergestellt nach einem der Ansprüche 1 bis 19 in Reaktionen zur Umwandlung von organischen Verbindungen.

21. Verwendung von Katalysatoren nach Anspruch 20, bei der die Reaktionen Dehydrierungsreaktionen gesättigter aliphatischer Kohlenwasserstoffe zu entsprechenden olefinischen Kohlenwasserstoffen sind.

22. Verwendung von Katalysatoren nach Anspruch 21, derart, das die aliphatischen gesättigten Kohlenwasserstoffe C3-C4 sind.

## Claims

1. A process for preparing a catalyst comprising at least one support, at least one metal from group VIII of the periodic table and at least one additional element M selected from the group formed by germanium, tin, lead, rhenium, gallium, indium and thallium, said process being **characterized in that** said metal M is introduced in an aqueous solvent in the form of at least one organometallic compound, soluble in an aqueous solvent, containing at least one carbon-M bond.

2. A process according to claim 1, in which the catalyst further contains at least one alkali or alkaline-earth metal.

3. A process according to claim 1 or claim 2, in which the catalyst further contains at least one metalloid.

4. A process according to any one of claims 1 to 3, in which the catalyst further contains at least one halogen or halogen-containing compound.

5. A process according to any one of claims 1 to 4, in which the group VIII metal is selected from iridium, nickel, palladium, platinum, rhodium and ruthenium, preferably from platinum and palladium.

6. A process according to any one of claims 1 to 5, in which element M is selected from germanium and tin.

7. A process according to any one of claims 1 to 6, in which the precursor of element M is selected from the group formed by hydroxides, halogen-containing compounds, carbonates and carboxylates of organic compounds of element M, compounds with general formula (R₁)ₓM(R₂)_{y} where x+y = the valency of metal M and where R₁ is selected from the group formed by alkyl, cycloalkyl, aryl, alkylaryl and arylalkyl functions, and R₂ is a function with formula CₐH_{b}R'_{c}, where R' represents a hydroxide, carboxylate, PO₃H or SO₃H function.

8. A process according to any one of claims 1 to 7, in which the precursor of element M is selected from the group formed by halogen-containing compounds, hydroxides, oxides, carbonates and carboxylates of organometallic compounds of element M.

9. A process according to any one of claims 1 to 7, in which the precursor of element M is selected from the group formed by carboxylates of organic compounds of element M.

10. A process according to claim 9, in which the precursor of element M is tributyltin acetate.

11. A process according to any one of claims 1 to 10, in which the group VIII metal, additional element M, optional halogen or halogen-containing compound, optional alkali or alkaline-earth metal, and optional metalloid are introduced successively or simultaneously.

12. A process for preparing a catalyst according to any one of claims 1 to 11, in which the following steps are carried out in any order:
• impregnating a support using an aqueous or organic solution of at least one group VIII metal, filtering, drying, calcining and reducing;
• impregnating the product obtained using an aqueous solution of at least one compound of additional element M, filtering, drying, optionally reducing, and calcining.

13. A process according to any one of claims 1 to 12, in which the support is impregnated with an aqueous solution of at least one compound of metal M, the volume of the solution being at least equal to the retention volume of the support, preferably higher than that volume.

14. A process according to any one of claims 1 to 13, in which the concentration of at least one metal M in the aqueous solution is in the range 0.01 to 25 mmol/l.

15. A process according to claim 14, in which the concentration of at least one metal M in the aqueous solution is in the range 0.5 to 20 mmol/l, preferably in the range 0.5 to 15 mmol/l.

16. A process for preparing a catalyst according to any one of claims 1 to 15, in which the pH of the aqueous solution of at least one compound of metal M is selected so as to be between 10 and 14, preferably between 10 and 12.

17. A process for preparing a catalyst according to any one of claims 1 to 11, in which additional element M is introduced during production of the support.

18. A process for preparing a catalyst according to claim 17, in which additional element M is introduced during synthesis of the support using a sol-gel type technique.

19. A process for preparing a catalyst according to claim 18, in which an aqueous solution of an organometallic compound of metal M is used to hydrolyse an organic solution of an alkoxy compound of a metal of the support in an alcoholic solvent, then heated to a temperature in the range 200°C to 800°C.

20. Use of catalysts prepared according to any one of claims 1 to 19 in organic compound transformation reactions.

21. Use of catalysts according to claim 20 in which said reactions are reactions for dehydrogenating saturated aliphatic hydrocarbons to the corresponding olefinic hydrocarbons.

22. Use of catalysts according to claim 21 in which said saturated aliphatic hydrocarbons are saturated C₃-C₄ aliphatic hydrocarbons.
